(19) Europäisches Patentamt / European Patent Office / Office européen des brevets

(11) EP 1 362 599 B1

(12) **EUROPEAN PATENT SPECIFICATION**

(45) Date of publication and mention of the grant of the patent:
**09.09.2009 Bulletin 2009/37**

(51) Int Cl.:
***A61K 47/48*** *(2006.01)*

(21) Application number: **02254597.4**

(22) Date of filing: **28.06.2002**

(54) **Conjugates of biodegradable aliphatic polyesters with peptides comprising a Tat-sequence**

Konjugate von biologisch abbaubaren aliphatischen Polyestern mit Peptiden enthaltend eine Tat-Sequence

Conjugués de polyesters biodégradables aliphatiques et des peptides comportant une séquence Tat

(84) Designated Contracting States:
**FR**

(30) Priority: **17.05.2002 KR 2002027328**

(43) Date of publication of application:
**19.11.2003 Bulletin 2003/47**

(73) Proprietor: **PACIFIC CORPORATION**
**Seoul 140-777 (KR)**

(72) Inventors:
- **Chang, Ih Seop,**
**102-1104, Dongbo Apt.**
**Yongin-si,**
**Kyunggi-do 449-759 (KR)**
- **Park, Ju Young,**
**202-502, Samsung 2-cha Apt.**
**Yongin-si,**
**Kyunggi-do 449-763 (KR)**
- **Nam, Yoon Sung,**
**901-204, Jukong Apt.**
**Yongin-si,**
**Kyunggi-do 449-993 (KR)**
- **Han, Sang Hoon,**
**2-203, Chungrok Apt.**
**Suwon-si,**
**Kyunggi-do 440-825 (KR)**

(74) Representative: **Harrison, Ivor Stanley et al**
**Withers & Rogers LLP**
**Goldings House**
**2 Hays Lane**
**London**
**SE1 2HW (GB)**

(56) References cited:
**WO-A-02/062396**

- **WENDER PAUL A ET AL: "The design, synthesis, and evaluation of molecules that enable or enhance cellular uptake: Peptoid molecular transporters" PROCEEDINGS OF THE NATIONAL ACADEMY OF SCIENCES OF THE UNITED STATES OF AMERICA, vol. 97, no. 24, 21 November 2000 (2000-11-21), pages 13003-13008, XP002265977 & ISSN: 0027-8424**
- **NAM YOON SUNG ET AL: "Intracellular drug delivery using poly(D,L-lactide-co-glycolide) nanoparticles derivatized with a peptide from a transcriptional activator protein of HIV-1." BIOTECHNOLOGY LETTERS, vol. 24, no. 24, December 2002 (2002-12), pages 2093-2098, XP0008026097 & ISSN: 0141-5492**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## BACKGROUND OF THE INVENTION

### 1. FIELD OF THE INVENTION

The present invention relates to conjugates of a biodegradable aliphatic polyester-based polymer with $Tat_{49-57}$ peptide or a peptide chain containing the $Tat_{49-57}$ peptide, and nanoparticles manufactured using the same. The term "$Tat_{49-57}$ peptide" as used herein refers to a peptide having the amino acid sequence of SEQ ID NO: 1.

### 2. DESCRIPTION OF THE RELATED ART

The usefulness of drug delivery systems using nanoparticles has recently been studied, and special attention is paid to studies for effective manufacturing of nanostructures through various synthetic routes of polymers. Among them, studies for attaching an adhesive molecule capable of recognizing a cell to the surface of a polymer nanoparticle in order to improve the adhesion efficiency of the polymer nanoparticle to living cells have been vigorously performed. For example, these studies include solubilizing non-soluble drug to enhance its bioavailability, heightening the intracellular absorptivity of macromolecular drugs such as proteins or genes, and introducing cell recognition molecules or cell adhesive molecules into polymer nanoparticles in order to specifically deliver drugs for curing terminal diseases such as cancer, to a target cell.

Examples of the cell recognition molecule or cell adhesive molecule include sugar moiety, antibodies, peptides, etc, and as strategies for applying these molecules to drug delivery, the concept of drug-polymer prodrug conjugates are widely used. In this sense, functional polymer into which water-soluble drug and cell recognition molecule, etc., are introduced was used to deliver a drug (H. Ringsdorf, J. Polymer Science (1977) 51, 155). Kopecek et al. demonstrates that drugs can be covalently conjugated to polymer chain by a peptide bond which is specifically cleaved by a lysosomal enzyme, after monoclonal antibodies, sugar moieties specific for liver cell, etc., are incorporated into the side chain of N-(2-hydroxypropyl)methacrylamide (HPMA) (R. Duncan et al., Biochim. Biophys. Acta. (1983) 755, 518; P. A. Flanagan et al., Biochim. Biophys. Acta. (1989) 39, 1125; D. Putnam and J. Kopecek, J. Adv. Polymn. Sci. (1995) 122, 55).

Particularly, since the 1990's, research for introducing a cell adhesive molecule into polymer nanoparticles has been earnestly active. T. Akaike et al. manufactured nanoparticles using phase separation of hydrophobic polymer and hydrophilic galactose in an aqueous solution, after synthesizing N-p-Vinylbenzyl-O-beta-D-galactopyranosyl-(1,4)-D-gluconamide by incorporating galactose molecule known as having high biding force with asialoglycoprotein receptor present on the surface of hepatocytes, liver parenchymal cell, into a hydrophobic polymer chain. As a result of in vitro and in vivo experiments for these nanoparticles, it was observed that the nanoparticles are not absorbed into non-parenchymal cells such as Kupper cell in cells constituting liver tissue, but are selectively absorbed into hepatocytes (S. Tobe et al., Biochem. Biophys. Res. Commun. (1992) 184, 225; K. Kobayashi et al., Macromolecules (1997) 30, 2016). Meanwhile, M. Hashida et al. synthesized cholesten-5-yloxy-*N*-(4-((1-imino-2-beta-D-thiomannosylethyl)amino)butyl) formamide (Man-C4-Chol), a cholesterol derivative, by introducing a mannose receptor which belongs to C-type lectin having strong binding force to macrophages, and improved the target activity towards macrophages by incorporating it into liposomes (P. Opanasopit et al., Biochim. Biophys. Acta. (2001) 1511, 134).

In addition to the endocytosis, as it was found that macromolecules are efficiently introduced into cells through the membranes of eukaryotic cells in an energy-independent manner by a peptide responsible for import cell signaling, researches for improving the intracellular permeability of macromolecules such as proteins, liposomes, nanoparticles, etc., using a membrane-permeable protein or a peptide present on the surface of viruses have been in rapid progress (M. Lindgren et al., Trends in Pharmacological Sciences (2000) 21, 99). It is highly estimated in that these researches enhance the pharmaceutical values of macromolecules such as curative proteins or genes, which had many limitations due to low biomembrane permeability and relatively short half life in vivo. S. R. Schwarze et al. reported that a cell membrane-permeable peptide is used for delivery of a drug with high molecular weight through the blood-brain barrier which is composed of a monolayer of endothelial cells (S. R. Schwarze et al., Science (1999) 285, 1569). C. Rousselle et al. performed the study for delivering a drug through the blood-brain barrier by binding D-penetratin (all amino acids are D-isomers) having the amino acid sequence of SEQ ID NO: 2 and a membrane-permeable peptide, SynB1 having the amino acid sequence of SEQ ID NO: 3 to doxorubicin, an anticancer agent (C. Rousselle et al., Molecular Pharmacology (2000) 57, 679).

Such a cell membrane-permeable peptide is mainly derived from proteins. These peptides are largely classified into three categories:

① Penetratin, a peptide derived from a homeodomain. It has the amino acid sequence of SEQ ID NO: 2. It was found in the homeodomain of Antennapedia which is a homeoprotein of Drosophila (A. Joliot et al., Proc. Natl. Acad.

Sci. U. S. A., (1991) 88, 1864). The term "homeoprotein" as used herein refers to a kind of transcription factor having about 60 amino acids which can bind to a DNA called a homeodomain.

② $Tat_{49-57}$ peptide present between amino acids 49-57 of Tat proteins, a transcription-activating protein of human immunodeficiency virus type-1 (HIV-1), which mediates acquired immune deficiency syndrome (AIDS). It has the amino acid sequence of SEQ ID NO: 1 (P. A. Wender et al., PNAS (2000) 97, 24, 13003-13008).

③ Peptides based on membrane translocating sequences (hereinafter, referred to as "MTS") or signal sequences. They are recognized by a receptor protein that helps place proteins produced by RNA in appropriate organelle membrane in vivo. It was also found that MTS bound to nuclear localization signal (hereinafter, referred to as "NLS") is accumulated within the cell nuclei after translocating across the cell membrane of several cells. The above mention was confirmed for MTS derived from the hydrophobic region of the signal sequence in, for example a Nuclear Transcription Factor kappa B (NF-κ B), Simian virus 40 (SV40) T-antigen or K-FGF bound to NLS peptide derived from Kaposi sarcoma fibroblast growth factor 1 (hereinafter referred to as "FGF"), human beta3 integrin, HIV-1 gp41, etc. (Y. Lin et al., J. Biol. Chem. (1996) 271, 5305; X. Lin et al., Proc. Natl. Acad. Sci. U. S. A. (1996) 93, 11819; M. C. Morris et al., Nucleic Acids Res. (1997) 25, 2730; L. Zhang et al., Proc. Natl. Acad. Sci. U. S. A. (1998) 95, 9184; Chaloin et al., Biochem. Biophys. Res. Commun. (1998) 243, 601; Y. Lin et al., J. Biol. Chem. (1995) 270, 14255).

When these peptides approach a cell after binding to cargo molecules, they act as an import signal and derive intracellular translocation of the cargo molecules. M. Rojas et al. conducted a study for glutathione-*S*-transferase-Grb2SH2 fusion protein (41 kDa) attached by the signal chain peptide, a transport peptide, having the amino acid sequence of SEQ ID NO: 4 to examine the intracellular effect on the EGF-stimulated signaling pathway of a fusion protein comprising Grb2SH2 domains (M. Rojas et al., Nature Biotech (1998) 16, 370). S. Fawell et al. attached the amino acids 32-72 of Tat protein to RNase A, in order to detect cellular cytotoxicity through the study about inhibition of protein synthesis by regulating the efficiency of internalization (S. Fawell et al., Proc. Natl. Acad. Sci. U. S. A. (1994) 91, 664). M. Rojas et al. attached the signal chain peptide having the amino acid sequence of SEQ ID NO: 4 to SHC Tyr 317 region (12 residues) in order to examine the effect on phosphorylation of Grb2 protein by the intracellular delivery of Grb2SH2 attached to peptides into SAA cells (M. Rojas et al., Biochem. Biophys. Res. Commun. (1997) 234, 675). J. Oehlke et al. attached the peptide, being an amphiphilic model peptide, having amino acid sequence of SEQ ID NO: 5 to SV40 large T antigen, in order to test the mobility of amphiphilic model peptide toward cells (J. Oehlke et al., Biochim. Biophys. Acta (1998) 1414, 127). L. Theodore et al. attached penetratin to PKC pseudo-substrate (14 residues), in order to inhibit the PKC activity of living neurocyte (T. Theodore et al., J. Neurosci. (1995) 15, 7158). S. Calvet et al. attached penetratin to FGF receptor phosphopetide (9 residues), in order to inhibit the receptor signal system of fibroblast growth factor (hereinafter, referred to as "FGF") in living neurocyte (S. Calvet et al., J. Neurosci. (1998) 18, 9751). M. C. Morris et al. attached MPG, a signal chain, to HIV natural primer binding site (36-mer), in order to detect intracellular delivery by a vector peptide (M. C. Morris et al., Nucleic Acid Res. (1997) 25, 2730). B. Allinquant et al. attached penetratin to APP antisense (25-mer), in order to control the decrease of amyloidal precursor protein for the study of the effect on growth of neural spine (B. Allinquant et al., J. Cell Biol. (1995) 128, 919). S. Dokka et al. attached a signal chain peptide having the amino acid sequence of SEQ ID NO: 4 to 10 oligo nucleic acid salts, in order to study the delivery of the oligo nucleic acid salts by combining them with the synthesized import signal (S. Dokka et al., Pharm. Res. (1997) 14, 1759). M. Pooga et al. attached penetratin and transportan to galanin receptor antisense (21-mer), in order to regulate galanin receptor levels and modify pain transmission in vivo (M. Pooga et al., Nature Biotech. (1998) 16, 857).

The term "Tat peptide" as used herein refers to a part of the Tat protein chain involved in the transcription of HIV, which mediates AIDS. The Tat protein is a transcriptional activation factor which is composed of 86 to 102 amino acids depending on virus strains. The Tat protein consists of three different functional domains: an acidic amino terminal region playing an important role in transactivation, a region corresponding to amino acids 22 to 37, in which zink-finger motif is contained and to which cysteine-rich nucleic acid can be attached, and a basic region corresponding to amino acids 49 to 58 responsible for nucleus permeability. Among them, the basic region is involved in the cell adhesion of protein independently of calcium ion (S. Ruben et al., J. Virol. (1989) 63,1; B. E. Vogel et al., J. Cell Biol. (1993) 121, 461). The Tat protein is secreted by living cells and intracellularly reinternalized, like the specific homeoprotein and herpes simplex virus type 1 protein VP22 (HSV-1 protein VP22), etc (B. Ensoli et al., J. Virol. (1993) 67, 277). The intracellular translocation is dependent on time and concentration, and is partly inhibited in case of low temperature. Further, because chloroquine or lysosomotropic agent prevents Tat protein from decomposing and stimulates its internalization in some cells, it is proposed that Tat protein can be internalized by endocytosis (A. D. Frankel and C. O. Pabo, Cell (1988) 55, 1189). However, from the fact that cell internalization of Tat protein has low dependency on temperature (D. A. Mann and A. D. Frankel, EMBO J. (1991) 10, 1733), an alternative mechanism, especially, competitive translocation mechanism is expected to exist. For example, when a cationic polymer such as heparin or dextran sulfate is added, the intracellular translocation of Tat protein is known to decrease. Such an effect seems to be caused by competition among charged molecules on the cell membrane. Meanwhile, it is also reported that the intracellular translocation of Tat protein is stimulated by the addition of a basic peptide such as protamine or a partial peptide of Tat protein (amino acids 38-58).

After intracellular internalization, the whole protein, or amino acids 1-86 or amino acids 37-72 of Tat protein is located in the cell nucleus. Particularly, amino acid sequence present on 48-60 is known as most effective region. Because this region contains a basic region of protein and NLS, the translocation of Tat protein into the cell or nucleus can be accomplished.

A. D. Frankel and C. O. Pabo from Johns Hopkins University Medical Center first noted the intracellular translocation of Tat protein. They found that 'Tat protein' produced by HIV virus had properties of NLS localizing to the nucleus as well as translocating into the cell membrane, and that these phenomena were promoted by a low concentration of 1 nmol chloroquine (A. D. Frankel, C. O. Pabo, Cell (1988) 55, 1189). Thereafter, upon searching for the peptide region in Tat peptide responsible for membrane permeability, it was found that a site consisting of six arginines, two lysines, and one glutamine plays an important role in cell permeability, and it has the amino acid sequence of SEQ ID NO: 1.

Recently, research results reporting translocation of polymers or proteins in vivo or in vitro using $Tat_{49-57}$ peptide or a peptide chain containing $Tat_{49-57}$ peptide have been reported. To date, it has been found that at least 10 peptides derived from Tat protein are translocated into different cells. It is also known that the intracellular translocation or internalization requires only a few minutes and is not highly sensitive to temperature. It is now known that the amino acid sequence of Tat protein effective for the intracellular delivery is a amino acids 49-57. A study for intracellular delivery by binding various cargo molecules to the above amino acid sequence has been performed. Examples of the molecules delivered by the cargo molecules include inhibitor of human papillomavirus type 16 (HPV-16), Cdk inhibitor $p27^{Kip1}$, $p16^{INK4a}$, capase-3 protein, ovalbumin to MHC class I pathway, beta-galactoxidase, etc. In addition, many studies for delivering molecules into cells have been performed using the arginine-rich amino acid sequence 48-60 of Tat protein. These molecules include DNA, macromolecules, proteins, drugs, drug delivery carriers, antigens, antibodies, hydrophilic polymers, inorganic nanoparticles, etc.

M. Lewin et al. at Massachusetts General Hospital, developed a superparamagnetic nanoparticle attached with a Tat peptide containing a short amino acid sequence 49-57 of Tat protein, which functions as a diagnostic substance to image the differentiation or distribution of precursor cells or stem cells in vivo with a high degree resolution. In this nanoparticle, 4 mer of amino acids -Gly-Tyr-Lys-Cys is attached to the carboxyl-terminal region for binding moiety of amino acids 49-57 of Tat protein, and a complex is manufactured by binding FITC, a fluorescent substance, and a nanoparticle having a diameter of 45 nm to the free -SH group of the cysteine (M. Lewin et al., Nature Biotech (2000) 18, 410). In the results, the effective internalization of Tat peptide-modified nanoparticles into haematopoietic cells and nerve cell precursors was confirmed from in vitro experiments using $CD34^+$ cells. Further, when Tat peptide-modified nanoparticles were injected intravenously into an immune deficient mouse, $CD34^+$ cells originated from bone marrow were confirmed by magnetic resonance imaging.

These results indicate that some amino acid sequences, and more specifically amino acids 49-57 of Tat protein, having cell internalization function, can be attached to a synthetic polymer capable of delivering drugs. To determine whether a large water-soluble polymer such as hydrogel can be intracellularly delivered using Tat peptide, J. Kopecek et al. from Utah University bound fluorescently labeled Tat peptide to N-(2-hydroxypropyl)methacrylamide (HPMA) copolymer and intracellular delivery experiments were performed using A2780 human ovarian carcinoma cells. As a result, it was confirmed that Tat peptide-attached HPMA is accumulated within the cells, particularly within nuclei, by a time-dependent non-endocytic pathway (A. Nori et al., 28th Proceed of International Symposium on Controlled Release Bioactive Materials, 2001, San Diego). These results show that even in the case of a copolymer having relatively high molecular weight, intracellular delivery can be performed through binding to Tat peptide. However, because the polymer used is a hydrogel soluble in an aqueous solution, it is required to conjugate a drug to be delivered with a polymer chain. Particularly, in the case of an insoluble drug, it is difficult to attain.

In addition to the above references, the following are pertinent: Wender, Paul A. et al: Proceedings of the National Academy of Sciences of the United States of America, vol. 97, no. 24, 21 November 2000, pages 13003-13008, discloses the synthesis and evaluation of molecules that enable or enhance cellular uptake, including polyguanidine derivatives of Tat peptide of amino acid sequence 49-57 (RKKRRQRRR). WO 02/062396 and WO 99/47173 A disclose delivery of therapeutic or diagnostic agents bound to a cell uptake promoter including Tat peptide of the same sequence and linked to specified polymers. Torchilin V. P. et al: Proceedings of the National Academy of Sciences of the United States of America, vol. 98, 17 July 2001, pages 8786-8791, indicates that a Tat peptide in the surface of liposomes affords their efficient intracellular delivery.

## SUMMARY OF THE INVENTION

The present invention provides a conjugate of a polymer with $Tat_{49-57}$ peptide having the amino acid sequene Arg-Lys-Lys-Arg-Arg-Gln-Arg-Arg-Arg or a peptide chain containing the $Tat_{49-57}$ peptide having said amino acid sequence, **characterised in that** the polymer comprises at least one biodegradable aliphatic polyester-based polymer selected from the group consisting of poly(D-lactic acid), poly(L-lactic acid), poly(D,L-lactic acid), poly(D-lactic acid-co-glycolic acid), poly(L-lactic acid-co-glycolic acid), and poly(D,L-lactic acid-co-glycolic acid), and copolymers produced

from the monomers corresponding to the above polymers.

## BRIEF DESCRIPTION OF DRAWINGS

The above and other objects, features and other advantages of the present invention will be more clearly understood from the following detailed description taken in conjunction with the accompanying drawings, in which:

Fig. 1 shows typical Fourier transform IR spectra of the conjugate (a) produced in Example 1 and pure poly(D,L-lactic acid-co-glycolic acid) (b);
Fig. 2 shows the size distributions of nanoparticles (c) manufactured in Example 4 and the nanoparticles (d) produced in Comparative Example 1;
Fig. 3 is a transmission electron microscopic image of the nanoparticles manufactured in Example 4;
Fig. 4 shows the results of MTT cytotoxicity assay for the nanoparticles manufactured in Example 4 and the nanoparticles manufactured in Comparative Example 1; and
Fig. 5a is a confocal laser scanning microscopic image showing the degree of intracellular translocation of nanoparticles manufactured in Comparative Example 1 at 37°C, and Fig. 5b is a confocal laser scanning microscopic image showing the degree of intracellular translocation of nanoparticles manufactured in Example 4 at 37°C.

## DETAILED DESCRIPTION OF THE INVENTION

The present invention provides conjugates of a biodegradable aliphatic polyester-based polymer with $Tat_{49-57}$ peptide or a peptide chain containing the $Tat_{49-57}$ peptide; and nanoparticles manufactured using the same. That is, the present invention provides conjugates of a biodegradable aliphatic polyester-based polymer with $Tat_{49-57}$ peptide, or conjugates of a biodegradable aliphatic polyester-based polymer with a peptide chain containing the $Tat_{49-57}$ peptide; and nanoparticles manufactured using the same.

The $Tat_{49-57}$ peptide consists of amino acids 49-57 of Tat protein, a transcription-activating protein of human immunodeficiency virus type-1 (HIV-1) which mediates acquired immune deficiency syndrome (AIDS), and has the amino acid sequence of SEQ ID NO: 1. At least one of the $Tat_{49-57}$ peptides or a peptide chains containing $Tat_{49-57}$ peptide is incorporated in the conjugate.

$Tat_{49-57}$ peptide or a peptide chain containing $Tat_{49-57}$ peptide is synthesized by solid phase peptide synthesis (SPPS) using amide 4-methylbenzhydrylamine hydrochloride (MBHA) resin with an ABI 433 synthesizer according to Fmoc(N-(9-fluorenyl)methoxycarbonyl)/tert-butyl method, but is not particularly limited thereto (M. Bodansky, A. Bodansky, The Practice of Peptide Synthesis; Springer: Berlin, Heidelberg, 1984, J.M. Stewart, J.D. Young, Solid Phase Peptide Synthesis, 2nd ed; Pierce Chemical Co: Rockford. IL, 1984).

The biodegradable aliphatic polyester-based polymer is a biocompatible polymer, and is required to decompose without induction of inflammation or immune reaction, and its decomposition product is required not to harm to the human body. The most common polymer to meet the requirements is a biodegradable aliphatic polyester-based polymer having lactic acid and glycolic acid as basic units, which is approved by the U.S. FDA. Representative examples of the biodegradable aliphatic polyester-based polymer include poly(D,L-lactic acid), poly(L-lactic acid) and poly(D-lactic acid) of the following formula 1, and poly(D,L-lactic acid-co-glycolic acid) of the following formula 2. The biodegradable aliphatic polyester-based polymer is at least one polymer selected from the group consisting of poly(D-lactic acid), poly(L-lactic acid), poly(D,L-lactic acid), poly(D-lactic acid-co-glycolic acid), poly(L-lactic acid-co-glycolic acid) and poly(D,L-lactic acid-co-glycolic acid), and copolymers produced from the monomers corresponding to the above polymers.

【Formula 1】

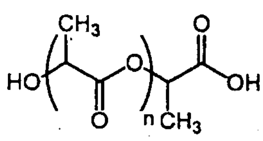

wherein
n is an integer of at least 2.

【Formula 2】

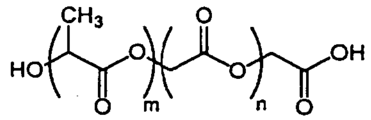

wherein
m and n are each, independently, integers of at least 2.

In the case of poly(D,L-lactic acid-co-glycolic acid) of formula 2, biodegradable polymer having various decomposition lifetimes can be obtained by controlling the ratio of monomers of lactic acid and glycolic acid, or changing the synthesizing pathway of polymer.

Such a biodegradable aliphatic polyester-based polymer has been used as a carrier for drug delivery or a suture for operation for a long time, and already demonstrated its biocompatibility. Meanwhile, the weight average molecular weight of the biodegradable aliphatic polyester-based polymer is in the range of 500 to 100,000, preferably 5,000 to 50,000 in order to achieve good effect on the production of nanoparticles, but is not particularly limited to these ranges,

$Tat_{49-57}$ peptide or a peptide chain (A) containing $Tat_{49-57}$ peptide, and the biodegradable aliphatic polyester-based polymer (B) may be constituted as A-B type or A-B-A type, but are not particularly limited thereto. First, carboxylic groups and hydroxyl groups present on both termini of the biodegradable aliphatic polyester-based polymer may be substituted with different functional groups so as to promote covalent bonding. Subsequently, the substituted termini of the polymer are reacted with termini of the $Tat_{49-57}$ peptide or the termini of peptide chain containing $Tat_{49-57}$ peptide to obtain the above constitution. For example, a conjugate of poly(D,L-lactic acid-co-glycolic acid) with the $Tat_{49-57}$ peptide or peptide chain containing $Tat_{49-57}$ peptide can be synthesized through the covalent bonding of poly(D,L-lactic acid-co-glycolic acid) substituted with maleimide and Tat peptide having thiol-substituted termini.

In the present invention, the covalent bonding can be formed by the addition of a base, a linker or a multiligand compound between the biodegradable aliphatic polyester-based polymer and the $Tat_{49-57}$ peptide or peptide chain containing $Tat_{49-57}$ peptide.

The present invention also relates to nanoparticles manufactured using the conjugate. At this time, the smaller the average size of the nanoparticles, the more preferable it is in view of the stability of colloid. For example, the average diameter of the nanoparticle is not more than 1,000 nm, preferably not more than 300 nm.

Membrane-permeability of Tat peptide can be taken effectively by exposing Tat peptide moieties on the surface of the nanoparticles according to the present invention, which results in enhanced intracellular permeability. Methods for manufacturing the nanoparticles according to the present invention include the following : sonicating after directly dispersing the polymer in an aqueous solution, extracting organic solvent with an excess of water or evaporating organic solvent after dispersing or dissolving the polymer in organic solvent, evaporating solvent under vigorous stirring condition by the use of a homogenizer or a high pressure emulsifier after dispersing or dissolving the polymer in organic solvent, dialyzing with an excess of water after dispersing or dissolving the polymer in organic solvent, adding water slowly after dispersing or dissolving the polymer in organic solvent, manufacturing using supercritical fluid, etc. (T. Niwa et al., J. Pharm. Sci. (1994) 83, 5, 727-732; C. S. Cho et al., Biomaterials (1997) 18, 323-326; T. Govender et al., J. Control. Rel. (1999) 57, 171-185; M. F. Zambaux et al., J. Control. Rel. (1998) 50, 31-40).

Examples of the organic solvents which can be used in the manufacture of the nanoparticles according to the present invention include acetone, dimethylsulfoxide, dimethylformamide, N-methylpyrrolidone, dioxane, tetrahydrofuran, ethyl acetate, acetonitrile, methyl ethyl ketone, methylene chloride, chloroform, methanol, ethanol, ethyl ether, diethyl ether, hexane or petroleum ether. At this time, the solvents can be used alone or in combination.

Further, the nanoparticles according to the present invention can be used as a drug delivery system with an improved bioavailability in vivo by introducing a specific drug therein.

Hereinafter, the present invention will now be described in more detail with reference to the following Examples, Comparative Examples and Experimental Examples, given by way of illustration.

**Example 1: Conjugate of poly(D,L-lactic acid-co-glycolic acid) and a peptide chain containing $Tat_{49-57}$ peptide**

A peptide chain containing $Tat_{49-57}$ peptide was synthesized by solid phase peptide synthesis (SPPS) using amide 4-methylbenzhydrylamine hydrochloride (MBHA) resin with an ABI 433 synthesizer according to Fmoc(N-(9-fluorenyl)methoxycarbonyl)/t-butyl method, and then purified by reverse high performance liquid chromatography (purity: greater than 90%). The molecular weight was determined to be 1846 by mass spectroscopy (Agilent 1100 series). These results confirmed that the peptide having the amino acid sequence of SEQ ID NO: 6, which contained chain contains

$Tat_{49-57}$ peptide and was added with a Gly-Tyr-Lys-Cys peptide consisting of 4 amino acids as a linker, was synthesized.

The conjugate of poly(D,L-lactic acid-co-glycolic acid) and the peptide chain containing $Tat_{49-57}$ peptide was synthesized through the covalent bonding of poly(D,L-lactic acid-co-glycolic acid) substituted with maleimide and the Tat peptide having thiol-substituted termini. The procedure is as follows: 80 ml of anhydrous 1,4-dioxane, 10 g of poly (D,L-lactic acid-co-glycolic acid) and 0.2 ml of triethylamine (TEA) were added to a reaction vessel, and the mixture was stirred to completely dissolve. To the reaction vessel a mixture of 1,3-dicyclohexylcarbodiimide (DCC) and N-hydroxysuccinimide (NHS) was added to activate the carboxylic groups in the main chain of the polymer. At this time, the molar ratio of carboxylic groups, dicyclohexylcarbodiimide and N-hydroxysuccinimide was 1:2:2. The mixture was stirred at room temperature, 1 atm under nitrogen atmosphere for 4 hours. 200 mg of hexamethylene diamine dissolved in 10 ml of anhydrous 1,4-dioxane was added to the reaction vessel and then stirred for 2 hours. The solution, which is obtained by filtration through a nylon filter with a pore size of 0.45 μm, the reaction mixture was subjected to precipitation with anhydrous diethyl ether, and the ether was removed to obtain a white solid. The solid reactant was again added to methylene chloride to dissolve the remaining reactant, reaction agents and byproducts. From this mixture, only the synthesized polymer was precipitated and collected. The above procedure was repeated three times to further purify the polymer. The purified polymer was dried under vacuum.

The polymer, poly(D,L-lactic acid-co-glycolic acid) having amine groups at the termini, thus obtained was dissolved in methylene chloride, and 1.5 times excess moles of N-succinimidyl 4-(4-maleimidophenyl)-butyrate was added thereto to derive maleimide to the termini of poly(D,L-lactic acid-co-glycolic acid). The synthesized polymer was precipitated with anhydrous diethyl ether, purified in accordance with the above precipitation method, and dried under vacuum. 3 ml of dimethylsulfoxide (DMSO) and 100 mg of polymer thus synthesized were added to a reaction vessel, and stirred to completely dissolve. 5 mg of peptide chain having the amino acid sequence of SEQ ID NO: 6 dissolved in 400 μl of reaction buffer (83 mM sodium phosphate buffer, 0.1 M EDTA, 0.9 M sodium chloride, 0.02% sodium azide, pH 7.2, with stabilizer) was added to the mixture, and then reacted at room temperature for at least 6 hours. After the reaction, the title compound obtained was purified by dialysis using cellulose membrane against distilled water, and lyophilized.

### Example 2: Conjugate of poly(D,L-lactic acid) and a peptide chain containing $Tat_{49-57}$ peptide

The title compound was produced in the same manner as in Example 1, except that poly(D,L-lactic acid) was used instead of poly(D,L-lactic acid-co-glycolic acid).

### Example 3: Conjugate of poly(L-lactic acid) and a peptide chain containing $Tat_{49-57}$ peptide

The title compound was produced in the same manner as in Example 1, except that poly(L-lactic acid) was used instead of poly(D,L-lactic acid-co-glycolic acid).

### Example 4: Nanoparticles using conjugate of poly(D,L-lactic acid-co-glycolic acid) and a peptide chain containing $Tat_{49-57}$ peptide

Nanoparticles according to the present invention were manufactured in accordance with phase inversion method. 100 mg of conjugate produced in Example 1 was dissolved in 10 ml of acetone, and then slowly added to 100 ml of phosphate buffer solution containing 0.5% w/v polyvinyl alcohol (PVA, 88% hydrolyzed, Mw of 25,000) with rapid stirring. Conjugate of poly(D,L-lactic acid-co-glycolic acid) and peptide chain containing fluorescently labeled $Tat_{49-57}$ peptide (5% by weight) in acetone was used to produce a fluorescently labeled polymer nanoparticle.

Meanwhile, the fluorescently labeled conjugate was produced in accordance with the following procedure. First, 10 g of the conjugate produced in Example 1 was subjected to esterification by 500 mg of dicyclohexylcarbodiimide and 300 mg of N-hydroxysuccinimide to activate carboxyl groups of the conjugate and then covalently bound to primary amine groups of fluorescein amine. The coupling reaction between the activated conjugate and the fluorescein amine was performed at room temperature under nitrogen atmosphere for 10 hours after adding 0.5 mg of triethylamine thereto. The dicycloureа precipitated as a byproduct was removed by filtration. The fluorescently labeled conjugate was precipitated with anhydrous diethyl ether, and purified in accordance with the above precipitation method.

### Example 5: Nanoparticles using conjugate of poly(D,L-lactic acid) and a peptide chain containing $Tat_{49-57}$ peptide

The title compound was manufactured in the same manner as in Example 4, except that the conjugate produced in Example 2 was used instead of the conjugate produced in Example 1.

**Example 6: Nanoparticles using conjugate of poly(L-lactic acid) and a peptide chain containing $Tat_{49-57}$ peptide**

The title compound was manufactured in the same manner as in Example 4, except that the conjugate produced in Example 3 was used instead of the conjugate produced in Example 1.

**Comparative Example 1: Nanoparticles of poly(D,L-lactic acid-co-glycolic acid)**

The title compound was manufactured in the same manner as in Example 4, except that pure poly(D,L-lactic acid-co-glycolic acid) was used instead of the conjugate produced in Example 1.

**Experimental Example 1: Confirmation of the conjugation of a biodegradable aliphatic polyester-based polymer and a peptide chain containing $Tat_{49-57}$ peptide.**

Confirmation of the conjugates according to the present invention was performed using an infrared spectrometer. Fig. 1 shows typical Fourier transform IR spectra of the conjugate (a) produced in Example 1 and pure poly(D,L-lactic acid-co-glycolic acid) (b). In the case of the conjugate (a) produced in Example 1, an amine-specific peak in the vicinity of 1656 $cm^{-1}$ was observed, in addition to an ester-specific peak in the vicinity of 1750 $cm^{-1}$. The presence of these peaks indicates that the peptide chain containing $Tat_{49-57}$ peptide was covalently conjugated to poly(D,L-lactic acid-co-glycolic acid).

**Experimental Example 2: Analysis of nanoparticles**

Surface potential of the nanoparticles manufactured in Example 4 and Comparative Example 1 was measured using Zetasizer 3000HS (Malvern, UK). Surface potential was - 7.8 mV for the nanoparticles manufactured in Comparative Example 1, while -0.9 mV for the nanoparticles manufactured in Example 4. This suggests that the peptide chain containing cationic lysine- and arginine-rich $Tat_{49-57}$ peptide orients toward the surface of nanoparticles, thereby increasing the surface potential.

The average particle sizes of the nanoparticles manufactured in Examples 4 to 6, and Comparative Example 1 were determined in accordance with a dynamic light scattering method (Zetasizer 3000HS, Malvern, UK). The scattering angle was fixed to an angle of 90° , and the experiment was carried out at 25°C. The hydrodynamic particle diameter was calculated by the Contin method. The results are listed in Table 1 and Fig. 2. As can be seen from the results, the average particle size of the nanoparticles (c) manufactured in Examples 4 to 6 is larger than that of the nanoparticle (d) manufactured in Comparative Example 1. This is thought to be resulting from the fact that the nanoparticles of pure poly (D,L-lactic acid-co-glycolic acid) have stronger hydrophobicity than the nanoparticles introduced by the peptide chain containing $Tat_{49-57}$ peptide, whereby forming a more compact nanostructure by their hydrophobic interaction.

[Table 1]

| | Conjugate used in the manufacture of nanoparticle | Average diameter of particle |
|---|---|---|
| Example 4 | Conjugate manufactured in Example 1 | 238 nm |
| Example 5 | Conjugate manufactured in Example 2 | 220 nm |
| Example 6 | Conjugate manufactured in Example 3 | 250 nm |
| Comparative Example 1 | Poly(D,L-lactic acid-co-glycolic acid) | 128 nm |

In addition, the shapes and the size distribution of the nanoparticles were observed using transmission electron microscopy (TEM, JEOL 2010). The test pieces were prepared by depositing one drop of 1 g/L nanoparticles dispersed in PBS onto a 100 mesh copper grid coated with carbon, and 1 minute after deposition, staining with 2% uranyl acetate solution. Fig. 3 is a transmission electron microscopic image of nanoparticles manufactured in Example 4. This shows that the nanoparticles have a discrete spherical morphology.

**Experimental Example 3: Cytotoxicity assay through cell culture**

The Cytotoxicity of the nanoparticles manufactured in Example 4 and Comparative Example I was evaluated using HaCaT (human corneous cell line) and HS-68 (human fibroblast cell line). The two cells were added to a cell culture medium (Dulbecco's modified Eagle's medium; hereinafter, referred to as "DMEM") supplemented with 1% by volume antibiotics (streptomycin, 10,000 μg/ml; penicillin, 10,000 IU/ml) and 10% by volume fetal bovine serum (hereinafter, referred to as "FBS") and incubated in an incubator filled with humidified air containing 5% $CO_2$ at 37°C. The two cells with 75% cell density in 96-well flat-bottomed plates were incubated with 1.5-50 μg/ml nanoparticles in 100 μl culture medium for 1 hour. Then, 10% by volume FBS was added thereto and incubated for an additional 48 hours. Thereafter, 3-(4,5-dimethylthiazol-2-yl)-2,5-diphenyl tetrazolium bromide (hereinafter, referred to as "MTT") and lactate dehydrogenase (hereinafter, referred to as "LDH") analyses were performed to evaluate cytotoxicity.

After MTT was added to cells incubated in 96-well plates to a final concentration of 500 μg/ml and the cells were incubated at 37°C for 4 hours, 100 μl of acidic isopropanol (0.04 N HCl in isopropanol) was added to each well and then mixed to dissolve dye material converted by living cells. ELISA plate reader (ELx800, Bio-TEK Instr. Inc.) was used to determine the absorbance of the converted dye material in each cell of 96-well plates at a wavelength of 550 nm. The MTT analysis standard curve was calculated by analyzing the relation of change in absorbance with respect to the number of living cells, after different numbers of cells were added to each well of 96-well plates, then incubating the cells in accordance with the above method, and followed by performing the MTT assay. The cytotoxicity assay of nanoparticles manufactured in Example 4 through the MTT assay was presented as % of living cells.

The amount of LDH eluted to the cell culture medium was measured using Cyto Tox 96 Non- Radioactive Cytotoxicity Assay kit (Promega, Madison, WI, USA). The debris of dead cells was separated by centrifuging the culture medium at a speed of 250 g for 4 minutes. After the centrifugation, 50 μl of supernatant was added to each well of 96-well plates. Subsequently, 50 μl of substrate solution was added thereto and left at room temperature for 30 minutes. To stop the reaction of the eluted LDH and substrate solution, 50 μl of 1.0 M acetic acid was added. The absorbance at 492 nm of the samples in each well was determined using an ELISA plate reader. The eluted LDH (%) was calculated using the following relation:

$$\text{Eluted LDH (\%)} = (\text{LDH eluted from the damaged cells (experimental group)} / \text{maximum LDH eluted from all cells after treating with lysis solution}) \times 100$$

Fig. 4 shows the results of MTT cytotoxicity assay of nanoparticles manufactured in Example 4 and nanoparticles manufactured in Comparative Example 1. These results show that there is no significant difference in eytotoxicity between the nanoparticles introduced by peptide chain containing the $Tat_{49-57}$ peptide and the nanoparticles containing no peptide chain.

**Experimental Example 4: Intracellular translocation of nanoparticles of a conjugate of poly(D,L-lactic acid-co-glycolic acid) and a peptide chain containing $Tat_{49-57}$ peptide**

Intracellular translocation of the nanoparticles manufactured in Examples 4 and 5 was confirmed using HaCaT cells, on which the nanoparticles were confirmed to have little or no effect on cell viability by Experimental Example 3, by the use of a confocal microscope (Radiance 2000/MP, Bio-rad). HaCaT cells grown in transparent 35 mm Delta T culture dishes (0.15 mm thick) were incubated in 1 ml DMEM culture medium supplemented with 1% by volume antibiotics and fluorescently labeled polymer nanoparticles (concentration = 50 μg/ml) for 1 hour under two conditions : ① air containing 5% $C0_2$ at 37°C, and ② air at 4°C. After culturing in two different conditions, respectively, the cells were washed three times with 1 ml of phosphate buffer solution, 1 ml DMEM culture medium was added to the culture dishes again, and the fluorescence of dyed cells was observed. The results are shown in Fig. 5.

Fig. 5a is a confocal laser scanning microscopic image showing the degree of intracellular translocation of nanoparticles manufactured in Comparative Example 1 at 37 °C (each scale interval is 10μm), and Fig. 5b is a confocal laser scanning microscopic image showing the degree of intracellular translocation of nanoparticles manufactured in Example 4 at 37°C (each scale interval is 10μm). As can be seen from the figures, in the case of nanoparticles of pure poly(D,L-lactic acid-co-glycolic acid), no intracellular translocation of the nanoparticles was observed; whereas in the case of the nanoparticles manufactured in Example 4, the nanoparticles were permeated through cell membranes and translocated into cells. Therefore, intracellular translocation of nanoparticles can be enhanced by introducing peptide chain containing $Tat_{49-57}$ peptide to nanoparticles.

We observed that intracellular permeability of $Tat_{49-57}$ peptide.can be enhanced by exposing Tat peptide moieties on the surface of the nanoparticles according to the present invention. Accordingly, nanoparticles according to the present invention can eliminate the disadvantages of polymer nanoparticles according to the prior art by covalently conjugating $Tat_{49-57}$ peptide or a peptide chain containing $Tat_{49-57}$ peptide, which has high biomembrane permeability, at the termini of polymer.

**Claims**

**1.** A conjugate of a polymer with $Tat_{49-57}$ peptide having the amino acid sequence Arg-Lys-Lys-Arg-Arg-Gln-Arg-Arg-Arg or a peptide chain containing the $Ta_{49-57}$ peptide having said amino acid sequence, **characterised in that** the polymer is at least one biodegradable aliphatic polyester-based polymer selected from the group consisting of poly(D-lactic acid), poly(L-lactic acid), poly(D,L-lactic acid), poly(D-lactic acid-co-glycolic acid), poly(L-lactic acid-co-glycolic acid) and poly(D,L-lactic acid-co-glycolic acid) and copolymers produced from the monomers corresponding to the above polymers, the conjugate optionally including a base, a linker or a multiligand compound added between the biodegradable aliphatic polyester-based polymer and the $Tat_{49-57}$ peptide or peptide chain containing the $Tat_{49-57}$ peptide.

2. A conjugate according to claim 1, in which the biodegradable aliphatic polyester-based polymer has a weight average molecular weight of from 500 to 100,000.

3. A conjugate according to claim 1 or claim 2, in which the $Tat_{49-57}$ peptide or the peptide chain (A) containing the $Tat_{49-57}$ peptide and the biodegradable aliphatic polyester-based polymer (B) are constituted as A-B type or A-B-A type.

4. A conjugate according to claim 3, in which the bond between A and B is a covalent bond.

5. A nanoparticle comprising the conjugate according to any preceding claim.

6. A nanoparticle according to claim 5, which has an average size diameter not more than 1,000 nm.

## Patentansprüche

1. Konjugat aus einem Polymer und einem $Tat_{49-57}$ Peptid mit der Aminosäuresequenz Arg-Lys-Lys-Arg-Arg-Gln-Arg-Arg-Arg oder einer Peptidkette, die das $Tat_{49-57}$ Peptid mit dieser Aminosäuresequenz umfaßt, **dadurch gekennzeichnet daß** das Polymer mindestens ein biologisch abbaubares, aliphatisches, auf einem Polyester basierendes Polymer ist, welches ausgewählt ist aus der Gruppe bestehend aus Poly(D-Milchsäure), Poly(L-Milchsäure), Poly (D,L-Milchsäure), Poly(D-Milchsäure-Co-Glykolsäure), Poly(L-Milchsäure-Co-Glykolsäure) und Poly(D,L-Milchsäure-Co-Glykolsäure) und Copolymeren, die aus den Monomeren hergestellt sind, welche den obigen Polymeren entsprechen, wobei das Konjugat wahlweise eine Base, ein Verbindungsmolekül oder eine Multi-Liganden-Verbindung umfaßt, welche zwischen dem biologisch abbaubaren aliphatischen, auf einem Polyester basierenden Polymer und dem $Tat_{49-57}$ Peptid oder der Peptidkette, die das $Tat_{49-57}$ Peptid umfaßt, eingefügt ist.

2. Konjugat nach Anspruch 1, in dem das biologisch abbaubare, aliphatische, auf einem Polyester basierende Polymer ein durchschnittliches Molekulargewicht von 500-100.000 aufweist.

3. Konjugat nach Anspruch 1 oder Anspruch 2, in dem das $Tat_{49-57}$ Peptid oder die Peptidkette (A), die das $Tat_{49-57}$ Peptid enthält, und das biologisch abbaubare, aliphatische, auf einem Polyester basierende Polymer (B) als A-B-Typ oder als A-B-A-Typ ausgebildet sind.

4. Konjugat nach Anspruch 3, in dem die Bindung zwischen A und B eine kovalente Bindung ist.

5. Nanopartikel, der das Konjugat nach einem der vorhergehenden Ansprüche umfaßt.

6. Nanopartikel nach Anspruch 5, der einen durchschnittlichen Durchmesser von nicht mehr als 1000 nm aufweist.

## Revendications

1. Conjugué de polymère avec le peptide $Tat_{49-57}$ ayant la séquence aminoacide Arg-Lys-Lys-Arg-Arg-Gln-Arg-Arg-Arg ou une chaîne peptide contenant le peptide $Tat_{49-57}$ ayant ladite séquence aminoacide, **caractérisé en ce que** le polymère est au moins un polymère à base de polyester aliphatique biodégradable sélectionné dans le groupe composé du poly(acide D-lactique), du poly(acide L-lactique), du poly(acide-D,L lactique) du poly(acide D-lactique-co-acide glycolique), du poly(acide L-lactique-co-acide glycolique) et du poly(acide D,L-lactique-co-acide glycolique) et des copolymères produits à partir des monomères correspondant aux polymères ci-dessus, ce conjugué incluant optionnellement une base, un liant ou un composé multiliant ajouté entre le polymère à base de polyester aliphatique biodégradable et le peptide $Tat_{49-57}$ ou la chaîne peptide contenant le peptide $Tat_{49-57}$.

2. Conjugué selon la revendication 1, dans lequel le polymère à base de polyester aliphatique biodégradable a un

poids moléculaire moyen de 500 à 100.000.

**3.** Conjugué selon la revendication 1 ou la revendication 2, dans lequel le peptide $Tat_{49-57}$ ou la chaîne peptide (A) contenant le peptide $Tat_{49-57}$ et le polymère à base de polyester aliphatique biodégradable (B) sont constitués selon le type A-B ou le type A-B-A.

**4.** Conjugué selon la revendication 3, dans lequel la liaison entre A et B est une liaison covalente.

**5.** Nanoparticule comprenant le conjugué selon l'une quelconque des revendications précédentes.

**6.** Nanoparticule selon la revendication 5 ayant un diamètre moyen ne dépassant pas 1.000 nm.

Fig. 1

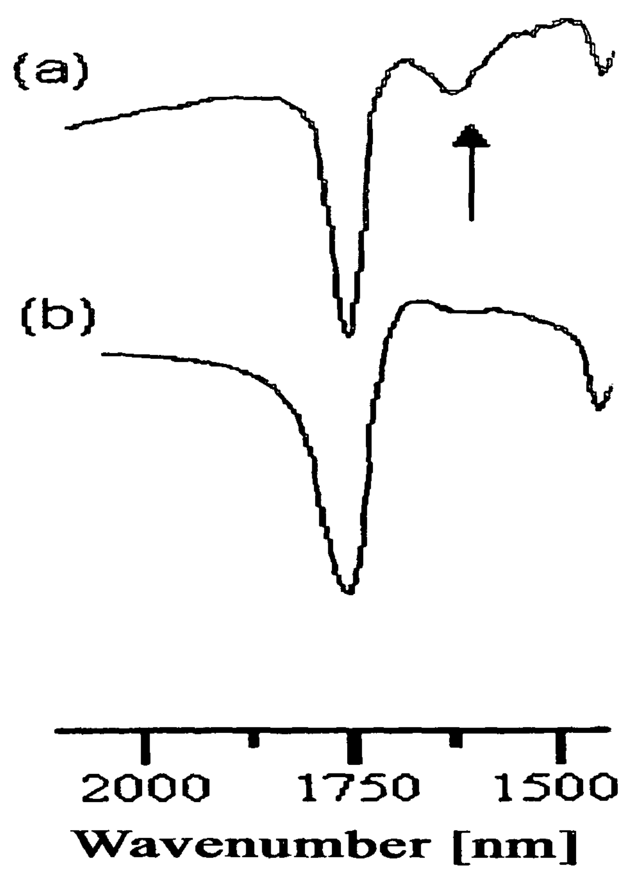

Fig. 2

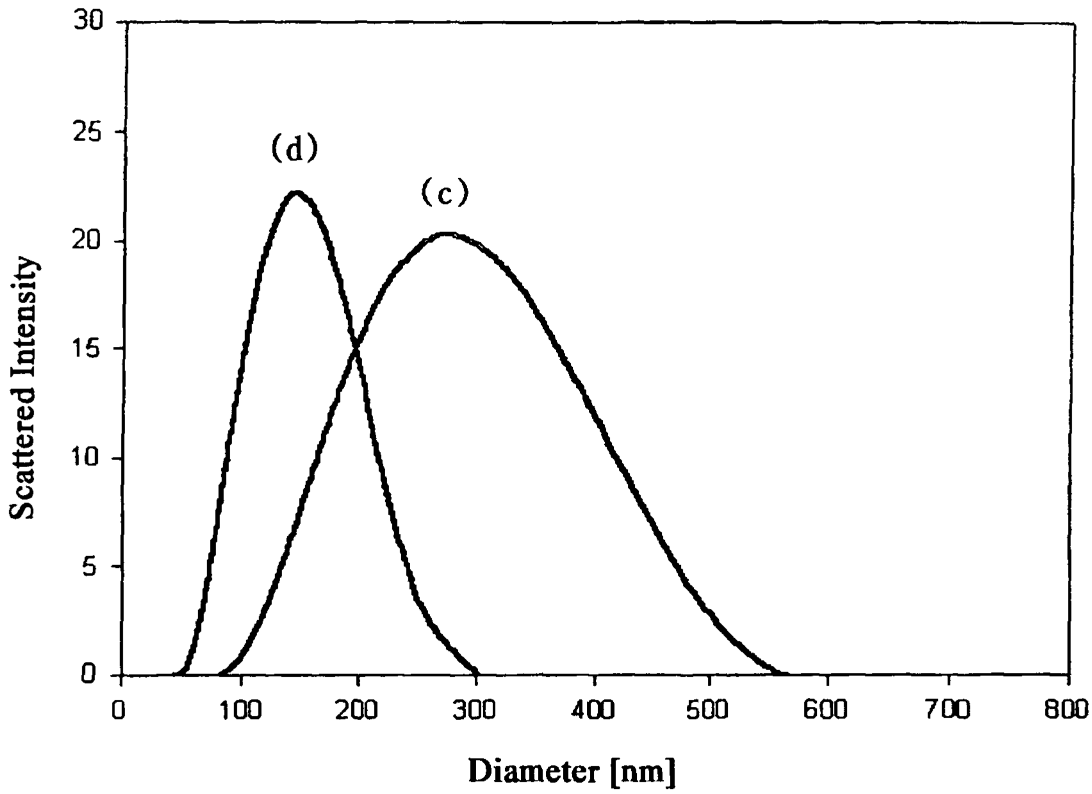
30
25
20
15
10
5
0
(d)
(c)
Scattered Intensity
0
100
200
300
400
500
600
700
800
Diameter [nm]

**Fig. 3**

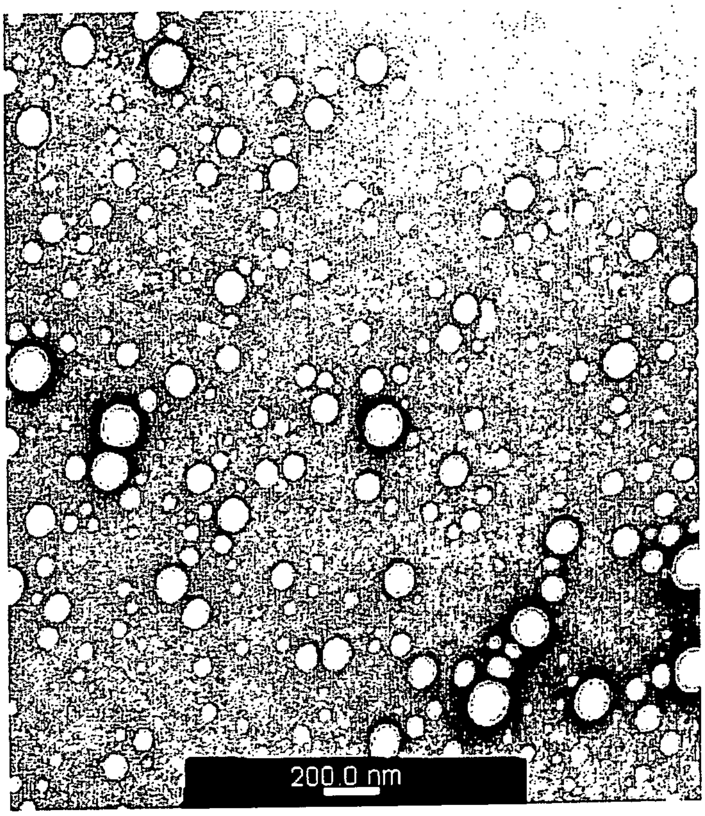

Fig. 4

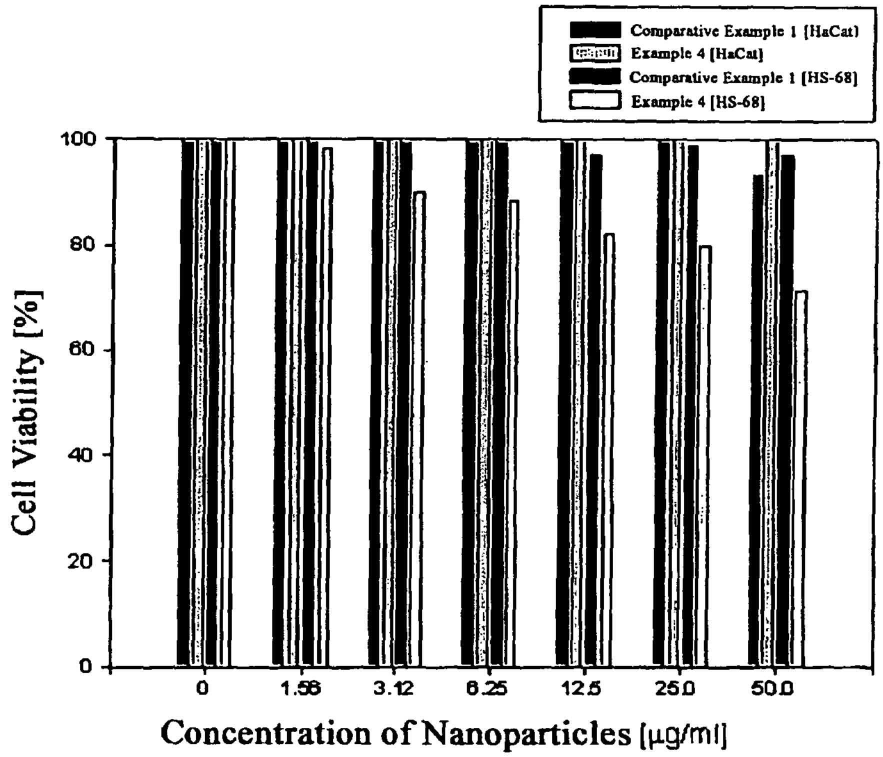
Comparative Example 1 [HaCat]
Example 4 [HaCat]
Comparative Example 1 [HS-68]
Example 4 [HS-68]
Cell Viability [%]
100
80
60
40
20
0
0
1.56
3.12
6.25
12.5
25.0
50.0
Concentration of Nanoparticles [μg/ml]

Fig. 5a

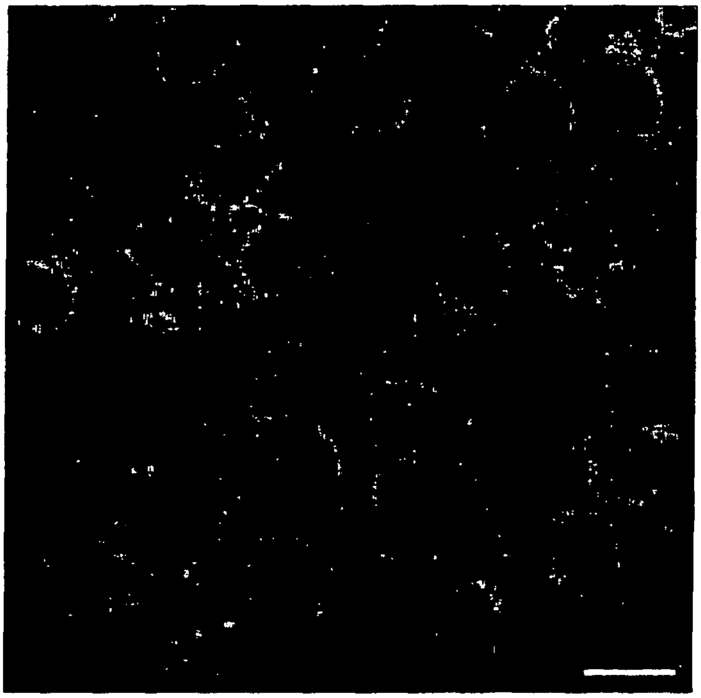

Fig. 5b

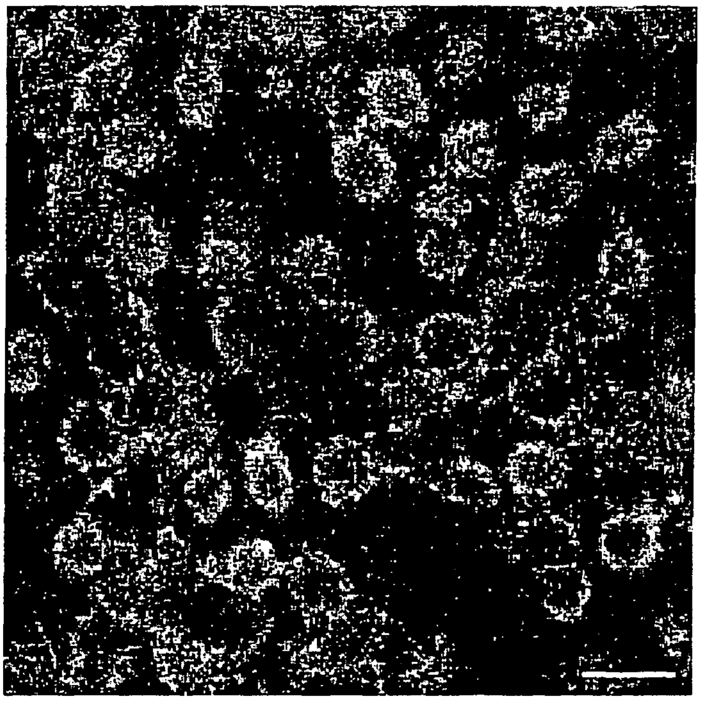

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02062396 A
- WO 9947173 A


### Non-patent literature cited in the description


- **H. Ringsdorf.** *J. Polymer Science,* 1977, vol. 51, 155
- **R. Duncan et al.** *Biochim. Biophys. Acta,* 1983, vol. 755, 518
- **P. A. Flanagan et al.** *Biochim. Biophys. Acta,* 1989, vol. 39, 1125
- **D. Putnam ; J. Kopecek.** *J. Adv. Polymn. Sci.,* 1995, vol. 122, 55
- **S. Tobe et al.** *Biochem. Biophys. Res. Commun,* 1992, vol. 184, 225
- **K. Kobayashi et al.** *Macromolecules,* 1997, vol. 30, 2016
- **P. Opanasopit et al.** *Biochim. Biophys. Acta,* 2001, vol. 1511, 134
- **M. Lindgren et al.** *Trends in Pharmacological Sciences,* 2000, vol. 21, 99
- **S. R. Schwarze et al.** *Science,* 1999, vol. 285, 1569
- **C. Rousselle et al.** *Molecular Pharmacology,* 2000, vol. 57, 679
- **A. Joliot et al.** *Proc. Natl. Acad. Sci. U. S. A.,* 1991, vol. 88, 1864
- **P. A. Wender et al.** *PNAS,* 2000, vol. 97 (24), 13003-13008
- **Y. Lin et al.** *J. Biol. Chem.,* 1996, vol. 271, 5305
- **X. Lin et al.** *Proc. Natl. Acad. Sci. U. S. A.,* 1996, vol. 93, 11819
- **M. C. Morris et al.** *Nucleic Acids Res.,* 1997, vol. 25, 2730
- **L. Zhang et al.** *Proc. Natl. Acad. Sci. U. S. A.,* 1998, vol. 95, 9184
- **Chaloin et al.** *Biochem. Biophys. Res. Commun,* 1998, vol. 243, 601
- **Y. Lin et al.** *J. Biol. Chem.,* 1995, vol. 270, 14255
- **M. Rojas et al.** *Nature Biotech,* 1998, vol. 16, 370
- **S. Fawell et al.** *Proc. Natl. Acad. Sci. U. S. A.,* 1994, vol. 91, 664
- **M. Rojas et al.** *Biochem. Biophys. Res. Commun,* 1997, vol. 234, 675
- **J. Oehlke et al.** *Biochim. Biophys. Acta,* 1998, vol. 1414, 127
- **T. Theodore et al.** *J. Neurosci.,* 1995, vol. 15, 7158
- **S. Calvet et al.** *J. Neurosci.,* 1998, vol. 18, 9751
- **M. C. Morris et al.** *Nucleic Acid Res.,* 1997, vol. 25, 2730
- **B. Allinquant et al.** *J. Cell Biol.,* 1995, vol. 128, 919
- **S. Dokka et al.** *Pharm. Res.,* 1997, vol. 14, 1759
- **M. Pooga et al.** *Nature Biotech.,* 1998, vol. 16, 857
- **S. Ruben et al.** *J. Virol.,* 1989, vol. 63, 1
- **B. E. Vogel et al.** *J. Cell Biol.,* 1993, vol. 121, 461
- **B. Ensoli et al.** *J. Virol.,* 1993, vol. 67, 277
- **A. D. Frankel ; C. O. Pabo.** *Cell,* 1988, vol. 55, 1189
- **D. A. Mann ; A. D. Frankel.** *EMBO J.,* 1991, vol. 10, 1733
- **M. Lewin et al.** *Nature Biotech,* 2000, vol. 18, 410
- **A. Nori et al.** *28th Proceed of International Symposium on Controlled Release Bioactive Materials,* 2001
- **Wender, Paul A. et al.** *Proceedings of the National Academy of Sciences of the United States of America,* 21 November 2000, vol. 97 (24), 13003-13008
- **Torchilin V. P. et al.** *Proceedings of the National Academy of Sciences of the United States of America,* 17 July 2001, vol. 98, 8786-8791
- **M. Bodansky ; A. Bodansky.** The Practice of Peptide Synthesis. Springer, 1984
- **J.M. Stewart ; J.D. Young.** Solid Phase Peptide Synthesis. Pierce Chemical Co, 1984
- **T. Niwa et al.** *J. Pharm. Sci.,* 1994, vol. 83 (5), 727-732
- **C. S. Cho et al.** *Biomaterials,* 1997, vol. 18, 323-326
- **T. Govender et al.** *J. Control. Rel.,* 1999, vol. 57, 171-185
- **M. F. Zambaux et al.** *J. Control. Rel.,* 1998, vol. 50, 31-40

[SEQUENCE LISTING]

<110> PACIFIC CORPORATION

<120> Conjugate of biodegradable aliphatic polyester with Tat49-57 peptide or peptide chain containing Tat49-57 peptide, and nanoparticle manufactured using the same

<130> 2002dp109

<160> 6

<170> Kopatent In 1.71

<210> 1
<211> 9
<212> PRT
<213> Human immunodeficiency virus type 1

<400> 1

Arg Lys Lys Arg Arg Gln Arg Arg Arg
1 5

<210> 2
<211> 16
<212> PRT
<213> Drosophila Antennapedia

<400> 2

Arg Gln Ile Lys Ile Trp Phe Gln Asn Arg Arg Met Lys Trp Lys Lys
1 5 10 15

<210> 3
<211> 18
<212> PRT
<213> Artificial Sequence

<220>
<223> peptide derived from SynBl of protegrins

<400> 3

Arg Gly Gly Arg Leu Ser Tyr Ser Arg Arg Arg Phe Ser Thr Ser Thr
1 5 10 15

Gly Arg

<210> 4
<211> 16
<212> PRT
<213> Artificial Sequence

<220>
<223> peptide derived from the src homology 2 (SH2) domain of Grb2

<400> 4

Ala Ala Val Ala Leu Leu Pro Ala Val Leu Leu Ala•Leu Leu Ala Pro
1 5 10 15

<210> 5
<211> 18
<212> PRT
<213> Artificial Sequence

<220>
<223> amphiphilic model peptide

<400> 5

Lys Leu Ala Leu Lys Leu Ala Leu Lys Ala Leu Lys Ala Ala Leu Lys
1 5 10 15

Leu Ala

<210> 6
<211> 14
<212> PRT
<213> Artificial Sequence

<220>
<223> peptide derived from Tat49-57 peptide

<400> 6

Gly Arg Lys Lys Arg Arg Gln Arg Arg Arg Gly Tyr Lys Cys
1 5 10